# FASCICULE DE BREVET EUROPEEN

(11) **EP 0 680 742 B1**
(45) Date de publication et mention de la délivrance du brevet: **05.02.2003**
(21) Numéro de dépôt: 95400442.0
(22) Date de dépôt: 01.03.1995
(51) Int. Cl.: A61K 7/043

(54) **Compositions cosmétiques à appliquer sur l'ongle**
Auf dem Nagel applizierende kosmetische Zusammensetzungen
Cosmetic compositions to be applied on the nail

(30) Priorité: 08.04.1994 FR 9404202
(43) Date de publication de la demande: 08.11.1995
(73) Titulaire: L'OREAL, 75008 Paris (FR)
(72) Inventeur: Mellul, Myriam, F-94240 l'Hay les Roses (FR); De la Poterie, Valérie, F-77820 Le Chatelet en Brie (FR)
(74) Mandataire: Dodin, Catherine

(56) Documents cités:
- EP-A- 0 143 480
- EP-A- 0 391 322
- EP-A- 0 418 469
- EP-A- 0 504 754
- EP-A- 0 506 300
- EP-A- 0 593 959
- EP-A- 0 628 304
- WO-A-93/18075
- US-A- 4 649 045

## Description

La présente invention a trait à une composition cosmétique à appliquer sur l'ongle comprenant comme agent filmogène une dispersion aqueuse de polymères.

Il est connu, par les documents EP-A-391 322 et EP-A-504 754, une composition cosmétique du type vernis à ongles, comprenant comme agent filmogène une dispersion aqueuse de polymère de polyuréthanne ou de copolymère polyuréthanne/acrylique styrène. Des compositions de vernis à ongles comprenant une dispersion aqueuse de polyuréthane et une dispersion acrylique sont aussi décrits dans les documents EP-A-143 480, EP-A-418 469, EP-A-593 959 et EP-A-628 304.
On a toutefois observé que toutes les dispersions aqueuses mentionnées dans ces documents ne présentaient pas une propriété filmogène correcte et ne permettaient donc pas d'obtenir, après étalement sur l'ongle, un film correct présentant une bonne tenue.

Des revêtements polymériques à base de dispersion aqueuse de polyuréthanes ont été décrits dans WO-A-93/18075.

Les dispersions aqueuses de polyuréthanes sont également connues pour former des gels avec une solution aqueuse de poly N-vinyl lactames, comme décrit dans EP-A-506 300.

Le but de la présente invention est de proposer des dispersions aqueuses permettant d'obtenir un vernis facilement applicable sur l'ongle, conduisant à un film de tenue correcte, brillant et pouvant se pelliculer.

Un objet de la présente invention est donc une composition cosmétique à appliquer sur l'ongle conforme à la revendication 1.

Un avantage de la présente invention est de permettre l'obtention sur l'ongle d'un film homogène, dur et brillant, adhérant bien sur l'ongle en le recouvrant parfaitement, tout en étant pelliculable.
Un autre avantage de l'invention est de permettre la réalisation d'un vernis à ongles contenant peu, voire pas, d'agent plastifiant ou d'agent de coalescence.

Par Tg, on entend dans la suite de la présente description, la température de transition vitreuse du polyuréthanne.
Par TMF, on entend dans la suite de la présente description, la température minimale de filmification du polymère, ou du mélange de polymères, radicalaire.

Un autre avantage de l'invention est de permettre la réalisation d'un vernis à ongles contenant peu, voire pas, d'agent plastifiant ou d'agent de coalescence.

Par Tg, on entend dans la suite de la présente description, la température de transition vitreuse du polyuréthanne.
Par TMF, on entend dans la suite de la présente description, la température minimale de filmification du polymère, ou du mélange de polymères, radicalaire.

Les pourcentages sont donnés en poids sauf indication contraire.
Les compositions selon l'invention sont caractérisées par le fait qu'elles comprennent une dispersion aqueuse d'un mélange de particules d'au moins un polyuréthanne et d'au moins un polymère radicalaire à groupement carboxylique, lesdits polyuréthannes et polymères radicalaires devant remplir certaines conditions physico-chimiques de manière à obtenir une filmification convenable desdites dispersions sur l'ongle.
On a en effet constaté que selon le type de polymères de polyuréthanne et/ou radicalaires que l'on emploie, il est ou non possible d'obtenir un film correct et pelliculable.
Seules les compositions selon l'invention permettent l'obtention, sur l'ongle, d'un film présentant les qualités nécessaires et satisfaisantes.

Afin de préparer les compositions selon l'invention, on prépare une dispersion aqueuse comprenant des particules de polyuréthanne et de polymère radicalaire à groupement carboxylique, par exemple par simple mélange d'une dispersion aqueuse de polyuréthanne et d'une dispersion aqueuse de polymère radicalaire, ou par formation directe d'une dispersion d'un mélange de particules de polyuréthanne et de polymère radicalaire.
La dispersion aqueuse de polyuréthanne peut être, par exemple, une dispersion aqueuse de polyuréthanne anionique, de polyester-polyuréthanne et/ou de polyéther-polyuréthanne, seul ou en mélange, pouvant présenter un taux de matière sèche de 20-40%.
Le polyuréthanne employé, ou le mélange de polyuréthanne, doit toutefois présenter une température de transition vitreuse (Tg) inférieure ou égale à environ 30°C.

La dispersion aqueuse de polymères radicalaires peut être choisie parmi toutes les dispersions aqueuses de polymères et/ou de copolymères acryliques, acryliques styrène et vinyliques, présentant de préférence un taux de matière sèche de 30-50%.
Lorsque la Tg du polyuréthanne employé est comprise entre 15°C et 25°C et que l'on choisit un polymère radicalaire ou un mélange de polymères radicalaires dont la TMF est inférieure ou égale à environ 30°C, on peut préparer, dans ce cas, une composition selon l'invention contenant 5-95% de dispersion de polyuréthanne et 5-95% de dispersion de polymères radicalaires.
Si l'on ne souhaite pas employer un polymère radicalaire, ou un mélange de polymères radicalaires, dont la TMF est inférieure ou égale à 30°C, il est nécessaire de ne pas dépasser un taux de 60% de dispersion de polymères radicalaires dans la composition.
Lorsque la Tg du polyuréthanne est comprise entre -15°C et -5 °C, il est nécessaire de ne pas dépasser un taux de 70% de dispersion de polymères radicalaires dans la composition.
On prépare de préférence des compositions cosmétiques contenant 10-60% de particules de polymères à l'état dispersé.
On peut ajouter dans la composition cosmétique des agents épaississants, par exemple de l'hydroxyéthylcellulose ou un dérivé cellulosique; un argile; un silicate ou un dérivé de silice; un polymère synthétique tel qu'un polymère radicalaire ou un polymère associatif de type polyuréthanne; une gomme naturelle telle que la gomme xanthane, en une proportion de 0,01-5%.
On peut également ajouter à la composition des additifs usuels tels que des agents d'étalement, des agents mouillants, des agents dispersants, des anti-mousses, des conservateurs, des filtres UV, des actifs, des agents hydratants, des alcools, des pigments minéraux ou organiques, seuls ou en mélange.

On obtient ainsi une composition cosmétique à étaler sur l'ongle qui présente de préférence, un taux de matière sèche de 10-60%, pouvant comprendre environ 1-40% de matière sèche de polyuréthanne et 1-50% de matière sèche de polymère radicalaire.
La composition cosmétique ainsi obtenue s'étale facilement sur l'ongle et permet l'obtention, après séchage, d'un film dur et brillant, présentant une bonne tenue sur l'ongle et pouvant se pelliculer facilement et d'un seul tenant.

Cette composition cosmétique peut être utilisée comme base pour vernis solvant de manière à permettre le démaquillage de l'ongle, par simple pelliculage, sans utiliser de solvant. Elle peut également, si elle contient des pigments, être utilisée comme vernis coloré, pelliculable.
Lorsqu'elle contient des actifs, la composition peut être employée comme base de soin pour l'ongle. Parmi les actifs envisageables, on peut citer les vitamines, la kératine et ses hydrolysats, la mélanine, les oligo-éléments, la glycérine et tout autre actif envisageable par l'homme du métier.

L'invention a également pour objet un procédé de traitement cosmétique de l'ongle caractérisé par le fait que l'on applique sur l'ongle une composition telle que définie ci-dessus.

L'invention est illustrée plus en détail dans les exemples suivants.

### Exemple 1

Plusieurs dispersions aqueuses de particules de polyuréthanne de Tg variables et de polymères radicalaires de TMF variables, dans des proportions variables, ont été préparées, par simple mélange d'une dispersion de polyuréthanne et d'une dispersion de polymères radicalaires et agitation pendant environ 30 minutes à température ambiante.
Les dispersions ainsi obtenues sont appliquées sur l'ongle et le film formé observé.

Polymères employés:
. polyuréthanne, Tg de -10°C: dispersion de polyuréthanne anionique aliphatique IW/010.1® de UCB, à 35% de matière sèche
. polyuréthanne, Tg de 21°C : dispersion de polyuréthanne anionique aliphatique IW/019.1® de UCB, à 35% de matière sèche
. polyuréthanne, Tg de 36°C: dispersion de polyuréthanne anionique aliphatique IW/028.1® de UCB, à 35% de matière sèche
. radicalaire, TMF de 12°C: dispersion d'acrylique styrène Joncryl 77® de Johnson, à 46% de matière sèche
. radicalaire, TMF de 35°C: dispersion d'acrylique Luhydran A848S® de BASF, à 45% de matière sèche
. radicalaire, TMF de 53°C: dispersion d'acrylique styrène Neocryl XK 63® de ICI, à 44% de matière sèche
. radicalaire, TMF de 86°C: dispersion d'acrylique styrène Joncryl 90® de Johnson, à 44% de matière sèche

On obtient les résultats suivants:
. extrait sec: taux de matière sèche de la dispersion avant application
. % : % de dispersion de polyuréthanne ou de dispersion de polymères radicalaires dans le mélange.

| Polyuréthanne | | Polymère radicalaire | | extrait sec | filmification |
|---|---|---|---|---|---|
| Tg (°C) | % | TMF (°C) | % | % | |
| -10 | 30 | 35 | 70 | 35 | oui |
| -10 | 20 | 86 | 80 | 35 | non |
| | 30 | | 70 | 35 | oui |
| | 50 | | 50 | 35 | oui |
| 21 | 20 | 12 | 80 | 35 | oui |
| | 70 | | 30 | 35 | oui |
| 21 | 20 | 35 | 80 | 35 | non |
| | 40 | | 60 | 35 | limite |
| | 50 | | 50 | 35 | oui |
| 21 | 20 | 53 | 80 | 35 | non |
| | 40 | | 60 | 35 | non |
| | 50 | | 50 | 35 | oui |
| 21 | 5 | 86 | 95 | 40 | non |
| | 40 | | 60 | 11 | non |
| | 50 | | 50 | 35 | oui |
| | 60 | | 40 | 16 | oui |
| 36 | 20 | 12 | 80 | 35 | non |
| | 40 | | 60 | 35 | non |
| | 60 | | 40 | 35 | non |
| | 70 | | 30 | 35 | non |
| 36 | 40 | 35 | 60 | 35 | non |
| | 80 | | 20 | 35 | non |
| 36 | 40 | 86 | 60 | 11 | non |
| | 60 | | 40 | 16 | non |
| | 70 | | 30 | 35 | non |
| | 80 | | 20 | 35 | non |
| | 90 | | 10 | 35 | non |

On constate donc que selon la Tg du polyuréthanne employé, on peut obtenir un film correct ou pas de film du tout.
Lorsque cette Tg est supérieure à environ 30°C, il n'est plus possible d'obtenir une bonne filmification sur l'ongle.

Lorsque la Tg est inférieure à 30°C mais supérieure à 0°C, on obtient un film satisfaisant lorsque la TMF du polymère radicalaire est inférieure ou égale à environ 30°C, ou lorsque la teneur en dispersion de polyuréthanne est d'au moins 40%.
De plus, lorsque la Tg du polyuréthanne est inférieure à environ 0°C, la teneur en dispersion aqueuse de polymères radicalaires doit être au maximum de 70%.

Cet exemple met en évidence le fait que tous les mélanges d'une dispersion de polyuréthanne avec une dispersion de polymère radicalaire ne permettent pas l'obtention d'un film correct, mais que seuls les mélanges tels que définis par l'invention permettent une filmification parfaite et l'obtention d'un vernis convenable.

### Exemple 2

On prépare un vernis à ongles ayant la composition suivante:
. dispersion aqueuse de polyuréthanne anionique aliphatique "IW/019.1®" (UCB), Tg < 30°C, 35% de matière sèche 56 g
. dispersion aqueuse de polymère acrylique anionique NEOCRYL XK 90(ICI)®, TMF : 18°C, matière sèche: 44,3% 19 g
. épaississant associatif polyuréthanne Ser AD FX 1100 (Servo) 0,8 g
. agent d'étalement KF 355A (Shin Etsu) 0,5 g
. pigments 1,5 g
. propylène glycol 0,2 g
. eau 22,2 g

On mélange tous les constituants à température ambiante et l'on agite ledit mélange pendant environ 30 minutes de manière à obtenir une composition homogène.
On obtient un vernis présentant un taux de matière sèche de 28%, aisément étalable sur l'ongle et permettant l'obtention, après séchage, d'un film brillant, de dureté et d'apparence satisfaisantes.
Ce film se pellicule facilement et d'un seul tenant.

## Revendications

1. Composition cosmétique à appliquer sur l'ongle comprenant comme agent filmogène une dispersion aqueuse de particules d'au moins un polyuréthanne et d'au moins un polymère radicalaire à groupement carboxylique, dans lequel :
. le polyuréthane a une température de transition vitreuse (Tg) comprise entre 15°C et 25°C, et le polymère radicalaire, ou le mélange de polymères radicalaires, a une température minimale de filmification (TMF) inférieure ou égale à 30°C, ou
. le polyuréthane a une température de transition vitreuse (Tg) comprise entre -15°C et -5°C, et le polymère radicalaire ou le mélange de polymères radicalaires, est présent à raison de 50-70% en poids.

2. Composition selon la revendication 1, **caractérisée par le fait que** la Tg du polyuréthane est comprise entre 15°C et 25°C et le polymère radicalaire, ou le mélange de polymères radicalaires, est présent à raison de 35-60% en poids.

3. Composition selon l'une quelconque des revendications précédentes, dans laquelle la dispersion aqueuse de polymères radicalaires à groupement carboxylique est choisie parmi les dispersions aqueuses de polymères et/ou copolymères acryliques, vinyliques et acryliques/styrène.

4. Composition selon l'une quelconque des revendications précédentes, dans laquelle la dispersion aqueuse de polyuréthanne est choisie parmi les dispersions aqueuses de polyuréthannes anioniques, de polyester-polyuréthanne et de polyéther-polyuréthanne.

5. Composition selon l'une quelconque des revendications précédentes, **caractérisée par le fait qu'**elle contient de 10 à 60 % en poids de particules de polymères à l'état dispersé.

6. Composition selon l'une quelconque des revendications précédentes, **caractérisée par le fait qu'**elle contient un additif choisi parmi les agents épaississants, les agents d'étalement, des agents mouillants, des agents dispersants, des anti-mousses, des conservateurs, des filtres UV, des actifs, des agents hydratants, des alcools, des pigments minéraux ou organiques, seuls ou en mélange.

7. Composition selon l'une quelconque des revendications 1 à 6, **caractérisée par le fait qu'**elle se présente sous la forme d'un vernis à ongles.

8. Procédé de traitement cosmétique de l'ongle, **caractérisé par le fait que** l'on applique sur l'ongle une composition selon l'une quelconque des revendications 1 à 7.

9. Utilisation, comme agent filmogène, d'une dispersion de particules d'au moins un polyuréthanne et d'au moins un polymère radicalaire à groupement carboxylique, **caractérisée par le fait que** :
. la température de transition vitreuse (Tg) du polyuréthane est comprise entre 15°C et 25°C, et le polymère radicalaire, ou le mélange de polymères radicalaires, a une température minimale de filmification (TMF) inférieure ou égale à 30°C, ou
. la température de transition vitreuse (Tg) du polyuréthane est comprise entre -15°C et -5°C, et le polymère radicalaire, ou le mélange de polymères radicalaires, est présent à raison de 50-70% en poids.
dans une composition cosmétique pelliculable à appliquer sur l'ongle.

10. Utilisation d'une composition selon l'une quelconque des revendications 1 à 8 pour obtenir, après application sur l'ongle, un film de bonne tenue, brillant et pelliculable.

## Patentansprüche

1. Kosmetische, auf die Nägel aufzutragende Zusammensetzung, die als Filmbildner eine wäßrige Dispersion von Partikeln aus mindestens einem Polyurethan und aus mindestens einem radikalischen Polymer, das Carboxygruppen aufweist, enthält, wobei
• das Polyurethan eine Glasübergangstemperatur (Tg) im Bereich von 15 bis 25 °C und das radikalische Polymer oder das Gemisch radikalischer Polymere eine Mindestfilmbildungstemperatur (MFT) von 30 °C oder darunter aufweist, oder
• das Polyurethan eine Glasübergangstemperatur (Tg) im Bereich von -15 bis -5 °C aufweist und das radikalische Polymer oder das Gemisch radikalischer Polymere in einem Anteil von 50 bis 70 Gew.-% enthalten ist.

2. Zusammensetzung nach Anspruch 1, **dadurch gekennzeichnet, daß** die Glasübergangstemperatur Tg des Polyurethans im Bereich von 15 bis 25 °C liegt und das radikalische Polymer oder das Gemisch radikalischer Polymere in einem Anteil von 35 bis 60 Gew.-% enthalten ist.

3. Zusammensetzung nach einem der vorhergehenden Ansprüche, wobei die wäßrige Dispersion radikalischer Polymere mit Carboxygruppe unter den wäßrigen Dispersionen von Acryl-, Vinyl- und Acryl/-Styrol-Polymeren und/oder Acryl-, Vinyl- und Acryl/Styrol-Copolymeren ausgewählt ist.

4. Zusammensetzung nach einem der vorhergehenden Ansprüche, wobei die wäßrige Polyurethandispersion unter den wäßrigen Dispersionen von anionischen Polyurethanen, von Polyester-Polyurethanen und von Polyether-Polyurethanen ausgewählt ist.

5. Zusammensetzung nach einem der vorhergehenden Ansprüche, **dadurch gekennzeichnet, daß** sie 10 bis 60 Gew.-% Polymerpartikel im dispergierten Zustand enthält.

6. Zusammensetzung nach einem der vorhergehenden Ansprüche, **dadurch gekennzeichnet, daß** sie einen Zusatzstoff enthält, der unter den Verdickungsmitteln, Spreitmitteln, Netzmitteln, Dispergiermitteln, Antischaummitteln, Konservierungsmitteln, UV-Filtern, Wirkstoffen, Hydratisierungsmitteln, Alkoholen, anorganischen oder organischen Pigmenten, einzeln oder im Gemisch, ausgewählt ist.

7. Zusammensetzung nach einem der Ansprüche 1 bis 6, **dadurch gekennzeichnet, daß** sie in Form eines Nagellacks vorliegt.

8. Verfahren zur kosmetischen Behandlung der Nägel, **dadurch gekennzeichnet, daß** auf die Nägel eine Zusammensetzung nach einem der Ansprüche 1 bis 7 aufgetragen wird.

9. Verwendung einer Dispersion von Partikeln aus mindestens einem Polyurethan und aus mindestens einem radikalischen Polymer, das Carboxygruppen enthält, als Filmbildner, die **dadurch gekennzeichnet ist, daß**
- die Glasübergangstemperatur (Tg) des Polyurethans im Bereich von 15 bis 25 °C liegt und das radikalische Polymer oder das Gemisch radikalischer Polymere eine Mindestfilmbildungstemperatur (MFT) von 30 °C oder darunter aufweist oder
- die Glasübergangstemperatur (Tg) des Polyurethans im Bereich von -15 bis
- 5 °C liegt und das radikalische Polymer oder das Gemisch radikalischer Polymere in einem Anteil von 50 bis 70 Gew.-% enthalten ist,
in einer auf die Nägel aufzutragenden, abziehbaren kosmetischen Zusammensetzung.

10. Verwendung einer Zusammensetzung nach einem der Ansprüche 1 bis 8, um nach dem Auftragen auf den Nagel einen Film zu erhalten, der eine gute Festigkeit und Glanz aufweist und abziehbar ist.

## Claims

1. Cosmetic composition for application to the nails, comprising as film-forming agent an aqueous dispersion of particles of at least one polyurethane and of at least one radical polymer containing carboxyl groups, in which:
• the polyurethane has a glass transition temperature (T_{g}) of between 15°C and 25°C, and the radical polymer or blend of radical polymers has a minimum filming temperature (MFT) below or equal to 30°C, or
• the polyurethane has a glass transition temperature (T_{g}) of between -15°C and -5°C, and the radical polymer or blend of radical polymers is present in the proportion of 50-70% by weight.

2. Composition according to Claim 1, **characterized in that** the T_{g} of the polyurethane is between 15°C and 25°C and the radical polymer or blend of radical polymers is present in the proportion of 35-60% by weight.

3. Composition according to either one of the preceding claims, in which the aqueous dispersion of radical polymers containing carboxyl groups is chosen from the aqueous dispersions of acrylic, vinyl and acrylic/styrene polymers and/or copolymers.

4. Composition according to any one of the preceding claims, in which the aqueous dispersion of polyurethane is chosen from the aqueous dispersions of anionic polyurethanes, of polyester polyurethane and of polyether polyurethane.

5. Composition according to any one of the preceding claims, **characterized in that** it contains from 10-60% by weight of polymer particles in the dispersed state.

6. Composition according to any one of the preceding claims, **characterized in that** it contains an additive chosen from thickening agents, spreading agents, wetting agents, dispersing agents, antifoams, preservatives, UV screening agents, active agents, hydrating agents, alcohols, and mineral or organic pigments, by themselves or as a mixture.

7. Composition according to any one of Claims 1 to 6, **characterized in that** it is in the form of a nail varnish.

8. Method for the cosmetic treatment of nails, **characterized in that** a composition according to any one of Claims 1 to 7 is applied to the nails.

9. Use, as film-forming agent, of a dispersion of particles of at least one polyurethane and of at least one radical polymer containing carboxyl groups, **characterized in that**:
• the glass transition temperature (T_{g}) of the polyurethane is between 15°C and 25°C, and the radical polymer or blend of radical polymers has a minimum filming temperature (MFT) below or equal to 30°C, or
• the glass transition temperature (T_{g}) of the polyurethane is between -15°C and -5°C, and the radical polymer or blend of radical polymers is present in the proportion of 50-70% by weight,
in a peel-off cosmetic composition to be applied to the nails.

10. Use of a composition according to any one of Claims 1 to 8 for obtaining, after being applied to the nail, a glossy film of good quality capable of being peeled off.
